# EUROPEAN PATENT APPLICATION

(11) **EP 4 468 307 A1**
(43) Date of publication of application: **27.11.2024**
(21) Application number: 23175264.3
(22) Date of filing: 25.05.2023
(51) Int. Cl.: G16H 30/40, G16H 40/63, G16H 50/20

(54) **WORKFLOW INTERFACES DRIVEN BY ARTIFICIAL INTELLIGENCE RESULTS**

(71) Applicant: Koninklijke Philips N.V., 5656 AG Eindhoven (NL)
(72) Inventor: YU, Bin, Eindhoven (NL); PLUYTER, Jon Ragnar, Eindhoven (NL); JACOBS, Igor, 5656AG Eindhoven (NL); MAVROEIDIS, Dimitrios, Eindhoven (NL); SCHUURKAMP, Gertjan Laurens, Eindhoven (NL); GEURTS, Lucas Jacobus Franciscus, Eindhoven (NL)
(74) Representative: Philips Intellectual Property & Standards

(57) **Abstract**

A system (98), comprising: a memory comprising instructions; and one or more processors configured by the instructions to: receive AI results of an applied AI model, wherein the AI results are related to a region of interest; receive a reliability metric associated with the AI results; and determine whether to provide the AI results or hide the AI results based on the reliability metric.

## Description

### FIELD OF THE INVENTION

The present invention is generally related to diagnostic imaging, and more particularly, to artificial intelligence-assisted, medical image processing.

### BACKGROUND OF THE INVENTION

In recent years, artificial intelligence (AI) has been extensively investigated as a tool for computer-aided diagnosis (CAD) in radiology. Thousands of AI solutions have been developed in research labs, and, to date, some of these perform on par, or even better than clinicians in some circumstances. For example, AI systems can accurately diagnose pneumonia on chest X-ray images, and detect breast cancer based on mammographies, and pulmonary nodules based on chest computed tomography (CT). Despite such performance, AI solutions face challenges in clinical practice adoption due at least in part to the poor fit of AI solutions in clinician workflows. Workflow integration is sometimes described as a greater barrier to using AI in healthcare than the lack of accuracy of AI findings. The introduction of Electronic Health Records is one example where poor human-centered development resulted in less-than-desired performance.

The combination of uncertainty of AI, and the life-critical, decision-making in the healthcare domain, drive the need for innovating on methodologies for human-centered design of AI in Radiology. While AI may increasingly outperform humans in some specific tasks, clinical decisions are not made without human intervention. Especially in healthcare, it is important that AI complements humans, such that humans and AI together form a collaborative system that is better than either of the two alone.

### SUMMARY OF THE INVENTION

The invention is defined by the independent claims. Dependent claims represent beneficial embodiments.

One object of the present invention is to improve upon existing systems in AI-assisted medical imaging-based detection and assessment of a region of interest. In a first aspect of the invention, a system is disclosed that receives AI results of an applied AI model, wherein the AI results are related to a region of interest, receives a reliability metric associated with the AI results, and determines whether to provide the AI results or hide the AI results based on the reliability metric. Deciding whether to incorporate or hide the AI-based results based on the reliability or confidence in the outcomes helps to improve the clinical workflow, and facilitates decisions on where to focus investigative efforts in assessing a condition of a subject.

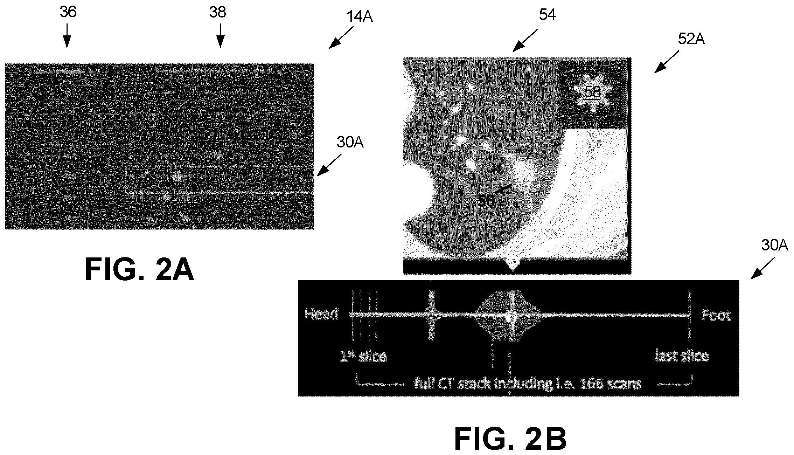

In one embodiment, the system is configured to adapt a first user interface according to a configuration of a user interface component selected from among plural configurations based on a scope of the AI results, wherein the user interface component comprises: a slider representing plural imaging slices, a marker corresponding to a location among the plural imaging slices of the region of interest, and a visualization superimposed on the marker and that is visually suggestive of characteristics of the region of interest from the AI results. In one embodiment, the navigation tool is embedded in a worklist as a user interface component, where the navigation tool is not only driven by the availability, certainty, and scope of AI results, but also serves as a navigation mechanism to other user interface components. In one embodiment, the navigation tool comprises a model that is embodied as user interface component(s) and image viewing area(s) (such as shown and described in association with FIGS. 2A-3D). The navigation tool adapts the first user interface, embodied as a worklist, according to one of a plurality of different configurations based on the richness or scope of information derived from the AI functionality. For instance, richness or scope of the AI results may be according to a range of results, where the AI results may range in scope from one to any combination of nodule detection (e.g., is a nodule detected or not), a prediction of malignancy risk, morphological characteristics (e.g., automated calculation of size, solidity, margins), and/or uncertainty/reliability metrics (which may be optional in some embodiments). The richness or scope of the AI results may be measurable, such as based on a threshold quantity of the AI results and/or threshold quality. The configuration of the user interface component(s) may thus vary depending on the richness of the AI results. The visualization may be in the form of an area chart that enables a clinician or user to readily determine certain characteristics of an abnormal growth region (or also referred to as a region of interest), using visualization features that are manifestations of the AI results, including size, shape and/or opacity that are suggestive of region of interest characteristics, including the size, shape, and severity (e.g., malignancy risk), enabling a natural integration of the navigation tool in the workflow of the clinician. In some embodiments, the visualization may be in the form of a bubble chart that is suggestive of fewer characteristics of the region of interest than is suggested by an area chart.

In one embodiment, the system is configured to provide a thumbnail image based on user input over the marker, the thumbnail image comprising an image of the region of interest, an identifying marker of the region of interest within an anatomical structure, and a visual cue suggestive of secondary characteristics of the region of interest. For instance, a user may hover a cursor (e.g., through the use of a mouse or other mechanism) over a bar or other marker of the slider on which the area chart is superimposed, which prompts the presentation of a thumbnail image and a visual cue that may be suggestive of size, margin shape, severity, and solidity of the region of interest. The visual cue provides a compact, easily readable visualization of region of interest characteristics, which again, improves clinician workflow.

In one embodiment, the system is configured to navigate to a second user interface based on user selection of the user interface component in the first user interface, wherein the one or more processors are further configured to adapt the second user interface according to one of plural assessment user interface configurations based on a scope of the AI results. The navigation tool provides the interactive and navigation features via the user interface component, such that user selection of, say, a visualization in the worklist, prompts an image slice containing the region of interest that may be further descriptive of the region of interest. The second user interface, which may be an image reading GUI (page) with an assessment panel, enables the clinician to dig down deeper into the slice containing the region of interest, as selected from the user interface component, providing an ease in the workflow in assessing information about the region of interest.

In one embodiment, the second user interface comprises an image reading page that includes an image involving the region of interest, and the region of interest, wherein the one or more processors are configured by the instructions to adapt the second user interface according to a first assessment user interface configuration selected from among the plural assessment user interface configurations based on a first scope among plural scopes of the AI results, wherein the first assessment user interface configuration further comprises: an identifying marker of the region of interest within an anatomical structure provided in the image; and a first assessment panel adjacent to the image, the first assessment panel comprising a first set of information corresponding to the first scope that is of greater scope than a rest of the plural scopes. The configuration for the assessment user interface or assessment panels are selected based on a varying scope or extent of information derived from the AI model to integrate well in providing a workflow that avoids information overload and/or deficiency.

These and other aspects of the invention will be apparent from and elucidated with reference to the embodiment(s) described hereinafter.

One of the advantages is that the clinical workflow can be improved.

Another advantage is that the system that will process, store and transmit the information in a more efficient way for subsequently presenting it to the user.

Another advantage resides in providing a radiology workstation providing for more efficient allocation of radiology examination reading tasks.

Another advantage resides in providing a radiology workstation with a more efficient user interface.

Further advantages of the present invention will be appreciated to those of ordinary skill in the art upon reading and understand the following detailed description. It will be appreciated that a given embodiment may provide none, one, two, or more of these advantages.

### BRIEF DESCRIPTION OF THE DRAWINGS

Many aspects of the invention can be better understood with reference to the following drawings, which are diagrammatic. The components in the drawings are not necessarily to scale, emphasis instead being placed upon clearly illustrating the principles of the present invention. Moreover, in the drawings, like reference numerals designate corresponding parts throughout the several views.
Fig. 1 is a schematic diagram of an example computer-aided diagnosis system, in accordance with an embodiment of the invention.
Figs. 2A-2B are screen diagrams of an example worklist with an integrated navigation tool via which a user can navigate to another user interface for an image slice on which a region of interest is found, in accordance with an embodiment of the invention.
Figs. 2C-2E are schematic diagrams that illustrate certain suggestive features of the user interface component(s), in accordance with an embodiment of the invention.
Figs. 3A-3B are screen diagrams of another example worklist with an integrated navigation tool via which a user can navigate to another user interface for an image slice on which a region of interest is found, in accordance with an embodiment of the invention.
Figs. 3C-3D are schematic diagrams that illustrate certain suggestive features of the user interface component(s), in accordance with an embodiment of the invention.
Fig. 4 is a screen diagram of an example image reading page according to a first configuration, in accordance with an embodiment of the invention.
Fig. 5 is a screen diagram of an example image reading page according to a second configuration, in accordance with an embodiment of the invention.
Fig. 6 is a screen diagram of an example image reading page according to a third configuration, in accordance with an embodiment of the invention.
Fig. 7 is a flow diagram of an example adaptive workflow user interface method, in accordance with an embodiment of the invention.
Fig. 8 is a schematic diagram of an example computing system, in accordance with an embodiment of the invention.

### DETAILED DESCRIPTION OF EMBODIMENTS

Disclosed herein are certain embodiments of a computer-aided diagnosis (CAD) system and method that are used to intelligently provide (e.g., present) artificial intelligence (AI)-based image detection and assessment results based on a reliability metric. In one embodiment, the CAD system comprises a user interface in the form of a worklist, wherein a navigation tool embedded therein is used to activate or enable different configurations of user interface components based on a comparison of the reliability metric to a threshold (e.g., to actually show or hide the AI results), and further based on a scope or richness of the AI results (e.g., for the different configurations). In one embodiment, the navigation tool comprises a model that is also embodied as user interface component(s) and image viewing area(s). The navigation tool adapts a user interface component(s) of the worklist with interactive visualizations, which are manifestations of the AI results, that efficiently suggest (e.g., by the appearance of the visualizations) characteristics of a region or regions of interest (e.g., abnormal growth, including pulmonary nodules, tumors, kidney stones, etc.) that have been detected by, among other mechanisms, the AI functionality. Indeed, the navigation tool itself adapts to the availability, reliability, and scope of AI results. The user interface components, also referred to herein in the singular (user interface component), and accordingly, also referred to herein as a user interface component(s) throughout, comprises a navigation slider corresponding to an imaging stack, which comprises a plurality of imaging slices. The navigation slider (or also simply referred to herein as a slider) comprises one or more markers that intersect the slider and that indicate a location within the stack of one or more regions of interest. The navigation tool may be used to enable or activate a visualization that may be superimposed (when activated) on the slider and marker, and which visually represents a region of interest with, for instance, an area chart or in some instances, a bubble chart, which is suggestive of certain characteristics of the region of interest. The navigation tool further enables or activates, in the UI component, a thumbnail image (or simply herein, thumbnail) corresponding to one or more of the slices containing the region of interest, the thumbnail prompted by user input (e.g., manipulating a cursor over the area chart). In some embodiments, the navigation tool enables user selection of the region of interest (e.g., via selection of the area/bubble charts) from the slider, which prompts a second user interface (e.g., an image reading page) adapted according to one of a plurality of different configurations based on the richness or scope of the AI results, and which may or may not show AI results based on the reliability metric (e.g., in comparison to the threshold). In effect, the CAD system adapts the user interfaces and interaction flow based on the availability, richness, and robustness of AI functionality. Hiding the results may mean any of the ways to conceal the results, which may not be shown to the user, which may include covering, masking, suppressing, curtaining, shadowing or otherwise concealing the results of the system. This may be through software and/or hardware configurations.

Digressing briefly, and referring to the detection and diagnosis of a region of interest in the form of pulmonary nodules as an illustrative example, radiologists often have a well-established diagnosis workflow, relying on years of image reading experience. If AI does not seamlessly fit into their workflows, or perhaps worse, if it creates additional complexity of information, radiologists may not embrace the technology. Both poor workflow fit, and information flow have been shown to hamper the uptake of AI. In existing CAD radiology image reading software, automatic detection and assessment of pulmonary nodules on chest computed tomography (CT) are empowered by AI technologies. By analyzing chest CT image data, the AI algorithms can provide radiologists a rich source of information about pulmonary nodules, including the location, size, shape, volume, and characteristics (e.g., severity (e.g., malignancy risk), margins, and solidity). Despite such rich nodule detection and analysis information generated by AI, there are still some problems regarding the proper integration of AI findings or results into radiology image reading flow, and information deficiency or overload may hinder the acceptance of AI functionality as a practical tool for pulmonary nodule assessment in clinical use. Meaningful presentation of AI-generated CAD information in the user interface of radiology image reading software and proper integration of AI into the image reading flow is a challenge in the emerging AI-empowered CAD systems.

Explaining further, some existing CAD systems reveal information deficiency or overload in radiology image reading for the assessment of pulmonary nodules. With regard to information deficiency, in nodule detection software, the AI-finding is often only marked with an AI-based bounding box around the nodule, whereas certain embodiments of a CAD system provide an indication of the certainty that the finding is a nodule. Also, in nodule malignancy risk prediction tools, often only an AI-based malignancy score is provided, whereas certain embodiments of a CAD system enhance any severity indicator with substantiating evidence (e.g., morphologic characteristics that increase the likelihood of a malignancy, such as large size, irregular, spiculated margins, etc.). As to information overload, often a multitude of AI-based nodule findings is listed per case, without smart filtering or a smart display where findings are relevant/certain enough to come forward in the reading workflow in contrast to certain embodiments of a CAD system. Also, unlike certain embodiments of a CAD system disclosed herein, existing systems do not take into account metrics associated with AI robustness (e.g. AI confidence) in the display of AI results to the radiologist, and/or AI results are often displayed as a number (e.g., rather than are translated into visualizations, visual cues or underlying interaction tools that can serve as functional UI elements for navigation).

Further, if AI-based results are not properly integrated and displayed in the radiologist reading flow, they can distract the radiologist or increase time spent per case. Currently, AI-based findings are often displayed as an add-on (e.g., an extra information field), whereas certain embodiments of a CAD system disclosed herein integrate AI results in a way that makes the reading more efficient (e.g., such as integrated into the slider of CT image stack). Explaining further, AI algorithm(s) may generate, for example, a malignancy risk prediction and derive nodule size. The navigation tool ingests the raw AI results and influences functional UI components for a worklist and/or image reading page. For instance, a slider within the worklist may be a functional UI component, where the navigation tool transforms AI results corresponding to a size of the nodule to a size of a visualization (e.g., size of an area or bubble chart), and the navigation tool transforms AI results corresponding to malignancy risk to an opacity of the visualization. In contrast, existing systems that use AI call the entire AI system, including its UI (User Interface), rather than at the granular level exhibited by operations of the navigation tool. That is, for existing systems, such adaptive representation is not accommodated. Many AI solutions existing today come with their own UI, and if external AI solutions are integrated into patient management applications, the AI solution often needs to be launched in a separate window or pop-up that opens that UI. This feature of existing systems becomes especially problematic if a nodule assessment system integrates AI models from different vendors, that all have their own UI. In existing systems, AI is not efficiently, or perceived as naturally integrated into, say, PACS. Static AI results may be displayed in a report, or as a new image series, whereas certain embodiments of a CAD system deeply integrates AI results into an image detection/assessment environment (e.g., such as in the slider), which improves workflow integration and thereby generates additional benefits (e.g., the findings can be used to navigate directly to more detailed or interesting finding, which can save time of the radiologist).

Having summarized certain features of a CAD system of the present disclosure, reference will now be made in detail to the description of a CAD system as illustrated in the drawings. While a CAD system will be described in connection with these drawings, with emphasis on detection and assessment of pulmonary nodules using CT imaging, there is no intent to limit it to the embodiment or embodiments disclosed herein. For instance, the CAD system may be used in other AI-based imaging detection and diagnosis applications in medical and/or research industries, including digital pathology or for abnormal growth detection/diagnosis in other (non-lung, such as kidney stones or uterine or ovary cysts) anatomical structures of a subject. As another example, in some embodiments, use of the reliability metric may be optional (e.g., showing the AI results regardless of the confidence and/or adaptation of the user interfaces based on other factors or considerations). Further, some embodiments may use non-AI algorithms to generate predictions, and/or the navigation tool may use non-AI results (e.g., clinician annotations of regions of interest) to visually represent or characterize regions of interest. Further, although the description identifies or describes specifics of one or more embodiments, such specifics are not necessarily part of every embodiment, nor are all of any various stated advantages necessarily associated with a single embodiment. On the contrary, the intent is to cover alternatives, modifications and equivalents included within the principles and scope of the disclosure as defined by the appended claims. For instance, two or more embodiments may be interchanged or combined in any combination. Further, it should be appreciated in the context of the present disclosure that the claims are not necessarily limited to the particular embodiments set out in the description.

To address one or more of the above problems, certain embodiments of a CAD system are described herein that integrate AI into an image reading workflow (e.g., radiology image reading workflow) to facilitate the detection and assessment (e.g., diagnosis) of pulmonary nodules on a chest CT. The CAD system relates to a comprehensive method that transforms the AI-generated pulmonary nodules detection/assessment results into interactive user interface (UI) components or elements that can serve as navigation tools, and generates adaptive workflows/interaction flows for a CAD-supported pulmonary nodule assessment depending on the AI output values. In general, the CAD system uses a reliability metric to determine whether to show or hide AI results, and comprises one or a combination of user interfaces that are adapted according to one of plural different configurations based on the richness or scope of information derived from the AI results (e.g., malignancy score, nodule size, type, morphology, etc.). Similarly, the workflow is adapted based on the richness of the AI results. In one embodiment, a user interface comprises a worklist for a radiologist, which incorporates or embeds a navigation tool that integrates three (graphical) user interface elements within a UI component or components. The UI components (or collectively, UI component) comprise a CT image stack slider, AI model output-based visualizations superimposed on the image stack slider, and a pop-up thumbnail image of the nodule that includes a visual cue suggestive of plural characteristics of the nodule. In some embodiments, a user interface comprises an image reading page that may be prompted or navigated to via selection of a visualization (e.g., area chart) superimposed on the slider (though other interactive mechanisms may be used to prompt the image reading page in some embodiments). The image reading page is adapted to one of plural different configurations based on the richness or scope of the AI results, and as indicated above, may or may not show AI results based on the reliability metric in some embodiments.

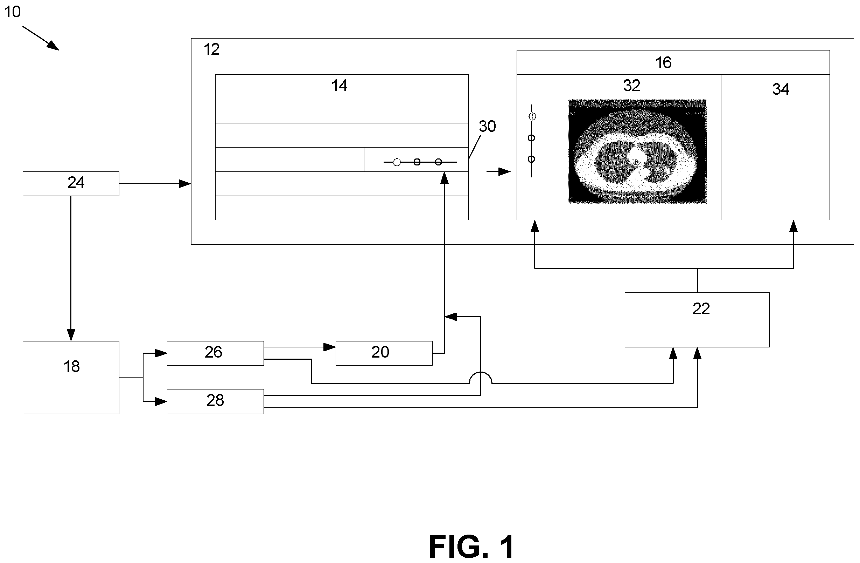

Having generally described certain features of an example CAD system, attention is directed to Fig. 1, which discloses an embodiment of a CAD system 10. The CAD system 10 comprises CAD-supported radiology reading software 12 (also simply referred to herein as CAD software 12), which includes a worklist graphical user interface (GUI) 14 and an image reading page GUI 16. In some embodiments, the CAD software 12 may include one or more additional components, such as reporting software and/or GUI. The worklist GUI 14 is also referred to herein as a worklist 14. The image reading page GUI 16 is also referred to herein as an image reading page 16. The CAD system 10 further comprises an AI model 18, a navigation tool 20, and an adaptive UI workflow component(s) 22, shown diagrammatically separate from the CAD software 12, though in some embodiments, one or more of these separately shown components may be incorporated into the CAD software 12. In other words, and as explained above, in one embodiment, the navigation tool 20 comprises a model that is embodied as user interface component(s) 30 (including image viewing area(s)).

In one example operation, data 24 is loaded into the CAD software 12 and displayed in the worklist 14 of the radiologist. The worklist 14 is shown in truncated view, but generally includes a multitude of data, including subject name and ID, exam type (e.g., chest CT), ordering physician, priority, status (e.g., scheduled, in progress, completed), assigned radiologist, exam location (e.g., department, facility), exam notes, and results or report status, with each row of the worklist 14 comprising a subject case/exam to be reviewed. With the AI-empowered CAD functionality of the CAD software 12, the data 24 (e.g., subject image data) is processed and analyzed by the AI model 18 to automatically detect and assess pulmonary nodules. For instance, data generated by the AI model 18 includes predicted results 26 about pulmonary nodules, and metrics 28 related to AI robustness (e.g., the confidence of predictions, or also referred to herein as a reliability metric). Note that in some embodiments, the predictions may be provided from non-AI algorithms existing in the art, or a combination of AI and non-AI algorithms. The AI-predicted nodule information (predicted results 26 and metrics 28) is received and configured for presentation by the navigation tool 20 as UI component(s) 30. In one embodiment, the navigation tool 20 adapts the worklist 14 according to different configurations based on the richness (e.g., scope) of the AI-generated nodule information. In some embodiments, the navigation tool 20 adapts the worklist 14 based on user-inputted information about the nodules (as opposed to, or in addition to, AI-generated predictions). For instance, the user-inputted information may include annotations of nodules or nodule features, such as inputted by a clinician, which may be used for adapting the UI component(s) 30. In some embodiments, one configuration of the worklist 14 includes not showing an AI-based UI component(s) 30 in the worklist 14 (e.g., the UI component(s) 30 is hidden from view) and/or based on unavailability of AI results. Digressing briefly, in general, existing systems do not include AI results in the worklist 14. Certain embodiments of a CAD system 10 incorporates AI results into the worklist 14 of the radiologist to help manage the workflow (e.g., prioritizing cases). In one embodiment, the AI results may be visible or not visible in each row of the worklist (e.g., based on if an AI-result is available, and based on the uncertainty of the AI results). For instance, if the uncertainty of the AI results is too high (e.g., in comparison to a threshold), the AI result may be omitted. In some instances, some detection modules in the CAD software 12 may not possess AI functionality, and hence AI results are not present in the worklist 14. For instance, the worklist 14 may comprise a variety of exams, for some of which an AI result may be available (e.g., based on the use of an AI-based lung nodule detection model), and for others, AI results are not available (e.g., no kidney tumor detection module is available).

The data 24 is provided to the AI model 18, and the AI model output or results may be used to drive functional UI components present in the worklist 14. In one embodiment, the navigation tool 20 comprises a model, and the model is embodied as the UI component(s) 30 in the worklist 14. The UI component(s) 30 comprises a slider and corresponding visualizations as described further below, where the functional or interactive components of the UI component(s) 30 are enabled or activated by the navigation tool 20 based on the AI results. In one embodiment, the decision by the navigation tool 20 to show or not show the UI component(s) 30 or certain features thereof is based on a comparison of the metric 28 associated with the predictions 26 to a threshold. For example, if a value of the metric 28 is less than a threshold value (e.g., as determined according to historical data, simulations, as defined by protocol, and/or as configured by a clinician), the UI component(s) 30, or certain feature of the UI component(s), is not shown. In effect, the metrics (associated with the predictions) of AI robustness enables a decision on the visibility of the navigation tool (e.g., manifested as the UI component(s) 30 or functional/interactive components of the UI component(s) 30) to prevent distracting or misleading the judgment of a radiologist that may be caused by an unreliable AI prediction (e.g., of low certainty). In some embodiments, the order of cases in the worklist 14 may be rearranged based on the metrics (and threshold). In some embodiments, the threshold value may also be interactive in the worklist 14, providing a clinician a mechanism to filter the worklist 14 based on the threshold value.

Continuing the description of an example operation, when the radiologist clicks on the UI component(s) 30 within the worklist 14, the software CAD software 12 prompts (e.g., navigates to) a display of the image reading page 16, which shows the specific image slice 32 among a CT stack represented by, and selected from, the UI component(s) 30. On the image reading page 16, a vertical version of the UI component(s) 30 and an AI assessment or results panel 34 is provided. Similar to the configurations provided by the navigation tool 20, the adaptive UI workflow 22 adapts the image reading page 16 according to one of a plurality of different assessment user interface configurations based on the richness or scope of the AI results, where the AI results may or may not be presented in the image reading page 16 based on a comparison of the AI results to the threshold as similarly described above. In other words, the configurations of the image reading page 16 are adaptive to the robustness and richness of the AI-generated nodule information. Note that in some embodiments, functionality of the navigation tool 20 and adaptive UI workflow 22 may be combined.

Fig. 2A illustrates the example worklist 14A (similar to worklist 14 of Fig. 1) and navigation tool-adapted user interface component(s) 30A (similar to user interface component(s) 30 of Fig. 1). The worklist 14A, again shown in truncated view, includes a plurality of rows of imaging-diagnostic results, and includes a severity column 36 and an overview column 38 of CAD nodule determination results. Additional data may be included within the worklist 14A, as explained above, including a subject identifier, date information, as well as other information to assist the radiologist in identification of the subject and/or nodule results. The overview column 38 may include the UI component(s) 30A provided by the CAD software 12 and adapted by the navigation tool 20, as shown in further detail in Fig. 2B. The UI component(s) 30A in this embodiment is adapted by the navigation tool 20 based on a scope or richness of the AI results. As explained above, the presentation of AI-based interactive or functional components within the UI component(s) 30A is based on a comparison of the AI results with a threshold, where it is assumed in this example that the comparison results in the presentation of AI results.

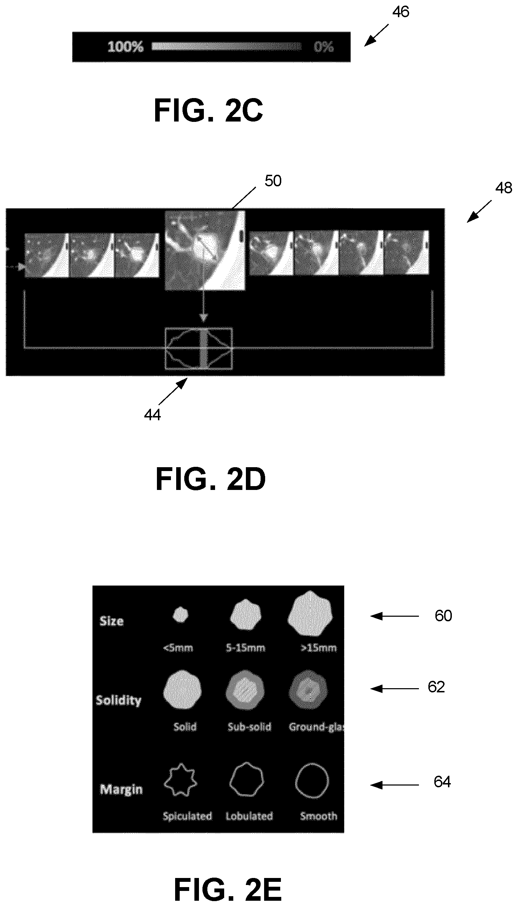

The UI component(s) 30A comprises a compact visualization of the overall AI findings/results on all pulmonary nodules that fits the limited space of a single row in the worklist 14A. With the advanced algorithms, the CAD software 12 and AI model 18 can detect, segment, and assess the lung nodule based on CT images. For instance, there might be a variety of nodule information generated by the AI model 18, including nodule position, segmentation, morphological characteristics that include size, solidity, margin, and malignancy prediction. The navigation tool 20 transforms this information derived from AI results into interactive/functional features or components manifested or embodied as the UI component(s) 30A. Stated otherwise, the face of the navigation tool 20 is the UI component(s) 30. The UI component(s) 30A visually shows and/or suggests different characteristics of the nodule, including one or any combination of nodule location, size, volume, and malignancy risk. In the depicted embodiment, the UI component(s) 30A comprises a slider 40 that visually represents a full CT stack comprising a plurality (e.g., 166 scans of the lung area ranging from head to foot) of CT slices, from which users may select a single CT slice to review. Markers 42, shown as bars that intersect the slider 40, are positioned along the slider 40 and indicate a central slice of the nodule. In effect, the markers 42 show a location of the nodule in the CT stack in, for instance, axial view (though other views may be shown). Note that reference to certain views presented by the features or visualizations associated with the UI component(s) 30A are for illustrative purposes, and in some embodiments, other views may be used (or, toggled between by a user in some embodiments). Nodule segmentation data is transformed by the navigation tool 20, based on the AI results, into a visualization that is suggestive of certain characteristics (e.g., morphological characteristics) of the region of interest (e.g., the nodule in this instance), and which enables navigation to other user interfaces or components. In one embodiment, the visualization comprises an area chart 44, which is superimposed on the marker 42. The area chart 44 shows a shape or contour (e.g., outline) of the nodules in, for instance, the coronal view (though other views may be presented). The height of the area chart 44 indicates a maximum diameter of the nodule in, for instance, the axial view. A width of the area chart 44 indicates the nodule size in, for instance, the axial or longaxis view. Therefore, the area chart 44 may show a volume of the nodule (e.g., when considered across one or more CT slices). An opacity of the chart area 44 indicates or suggests a severity (e.g., the malignancy risk or probability) according to a range (e.g., 0 - 100%) of opacity 46, as shown in Fig. 2C. An example CT stack 48 is shown in Fig. 2D for further illustration of what the UI component(s) 30A suggests or conveys, with the size of the chart area 44 for a selected slice 50 further illustrated amongst slices of the CT stack 48.

Note that in some embodiments, the level of granularity provided by the visual features of the area chart 44 may be omitted or lessened (e.g., by using a different form of visualization) for the worklist 14. For instance, the worklist 14 may be primarily used to prioritize and select cases for review, and thus a more extensive scope of information about the region of interest (e.g., nodule) may be reserved for the image reading page 16, where it may be more relevant to learn of, for instance, the image slice with the largest nodule diameter. In some embodiments, the area chart 44 may be replaced with an alternative, less-suggestive visualization, or omitted, based on unavailability of sufficient screen realestate in the worklist 14.

A presentation of a thumbnail 52A is prompted by user input over the area chart 44. For instance, a user may hover a mouse cursor over the area chart 44, which causes the presentation of the thumbnail 52A. The thumbnail 52A comprises an image of an anatomical region 54 and an identification 56 of the nodule within the anatomical region 54. Though the identification 56 is depicted as a dashed boundary surrounding an outline of the nodule, in some embodiments, other mechanisms of visualization may be used, including a translucent, colored highlight superimposed on the nodule, an arrow pointing at the nodule, bounding box, etc. Note that the identification 56 may differ in appearance from the area chart 44. For instance, the identification 56 may be of a different view than that represented by the area chart 44, and/or the identification 56 may be configured to merely show location within the image slice as opposed to identically or nearly identically circumscribing the nodule. Explaining further, one purpose of the identification 56 may be to merely pinpoint the location of the nodule within the image. When the nodule is segmented in the image, the circumference or outline may also be shown. Even so, the shape of the nodule between the area chart 44 and what is identified via the identification 56 may be different because the image of the anatomical region 54 (e.g., lung) may be a cross sectional plane and the outline reflects the nodule boundaries in that plane, whereas in the slider 40 and the area chart 44, the shape is more a reflection of a maximum nodule diameter in a set of consecutive slices of the lung that go from head to feet.

The thumbnail 52A further comprises a visual cue 58, which is a visualization, superimposed (shown in the upper right corner, though the location may be in other areas of the thumbnail 52A) on the image of the anatomical region 54, and that suggests characteristics of the nodule. For instance, and referring to Fig. 2E, the visual cue 58 may suggest various morphological characteristics, including a size 60 (e.g., < 5mm, 5-15 mm, > 15mm, though other values or ranges may be used in some embodiments), solidity 62 (e.g., solid, sub-solid, ground-glass), and/or margin 64 (e.g., spiculated, lobulated, smooth). The characteristics of the nodule presented by the visualizations are based on the richness or scope of the AI results. The richness or scope of the AI results may be according to a range of results. For instance, the AI results may comprise one or any combination of nodule detection (e.g., is a nodule detected or not), a prediction of malignancy risk, morphological characteristics (e.g., automated calculation of size, solidity, margins), and/or uncertainty/reliability metrics (which may be optional in some embodiments). The configuration of the UI component(s) 30A may thus vary depending on the richness of the AI results.

As described below, the image reading page 16 may be prompted (e.g., navigated to) based on user input on the slider 40. For instance, a user may click on the area chart 44, which prompts the display of the image reading page 16.

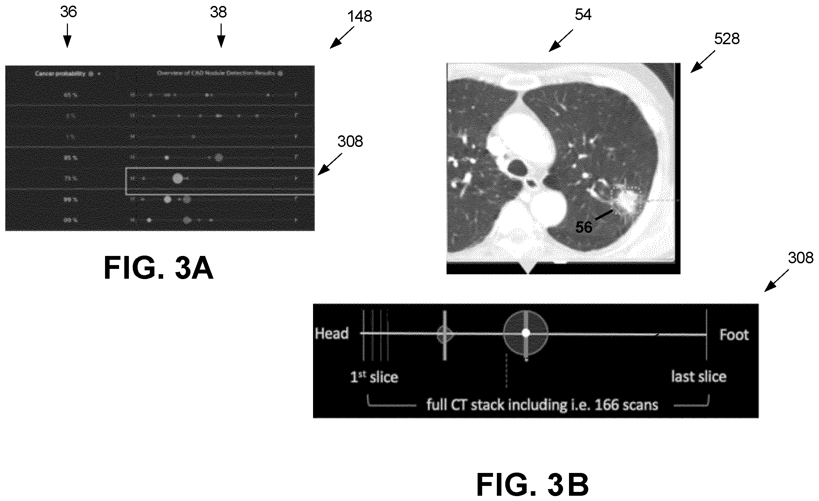

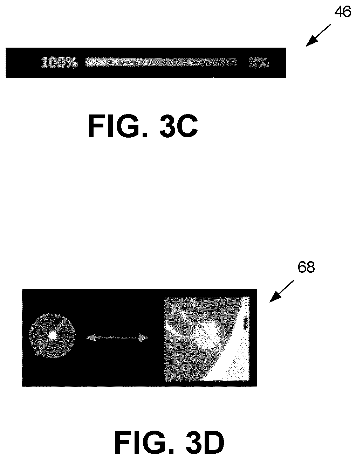

When the AI results are of a lesser scope or richness (e.g., than that provided for the configuration of Figs. 2A-2B), the CAD system 10 adapts the worklist 14 according to a different configuration. Referring to Figs. 3A-3B, shown is an example worklist 14B and navigation tool embodied as a user interface component(s) 30B. Similar to the worklist 14A of Fig. 2A, the worklist 14B includes a plurality of rows of imaging-diagnostic results (shown in partial or truncated form as similarly described above), and includes a severity column 36 and an overview column 38 of CAD nodule determination results. Additional data may be included within the worklist 14B, as similarly described for worklist 14A above. The overview column 38 may include the UI component(s) 30B adapted by the navigation tool 20, as shown in further detail in Fig. 3B. The UI component(s) 30B in this embodiment is adapted by the navigation tool 20 based on a scope or richness of the AI results. In some embodiments, the determination on whether to present the AI results is based on comparison to the threshold, or the availability of AI results. Note that elements of the worklist 14A and UI component(s) 30A that are the same or similar to the worklist 14B and UI component(s) 30B are designated with the same reference numbers, and discussion of the same is omitted here for brevity. A focus of discussion in the following description is directed to the differences between the elements of the worklist 14A and UI component(s) 30A, and the worklist 14B and UI component(s) 30B. In the worklist 14B and UI component(s) 30B, bubble charts 66 replace the area charts 44. That is, the nodules are visualized by a bubble charts 66 instead, which indicates or suggests a size of the nodule. For instance, a maximum nodule diameter derived from the segmented nodule determines the size of the bubble. A severity (e.g., malignancy risk) of the nodule is represented by the opacity of the filling color, as further illustrated by the opacity range 46 in Fig. 3C. The diameter of the bubble (circle) corresponds to a maximum diameter (of the nodule in, for instance, the axial view, though as described above, other views may be used) or volume of the nodule, as reflected by the image slice 68 shown in Fig. 3D. Instead of visual representation of the fuller nodule characteristics, as shown by the configuration in Figs. 2A-2B, the configuration shown in Figs. 3A-3B only suggests the nodule size, and location, and severity (e.g., via opacity). The thumbnail 52B, which is similarly triggered as with the thumbnail 52A in Fig. 2B, reveals the identification 56 of the nodule on the image of the anatomical region 54, which is suggestive or the size and location of the nodule. Note that the identification 56 may differ among different configurations based on the scope of the AI results (e.g., a bounding box may be used where features of the nodule are missing from the AI results). Notably no visual cue (e.g., visual cue 58 in Fig. 2B) is presented. Note that the bubble chart 66 is in the form of a circle, though in some embodiments, may be of another geometrical shape. Also similar to the configuration in Fig. 2B, when a user selects the bubble chart 66, the CAD system 10 (via the navigation tool functionality) provides the image reading page 16 that also has a configuration based on the richness or scope of the AI results, and for which the AI results may or may not be shown based on the reliability metric.

In some embodiments, the worklist 14 does not necessarily display all information generated by the AI model 18. For instance, there may be selective presentation corresponding to available metrics (e.g., all data for available metrics, or only the metrics relevant to prioritize and select the case and navigate to the slice on which the nodule is visible).

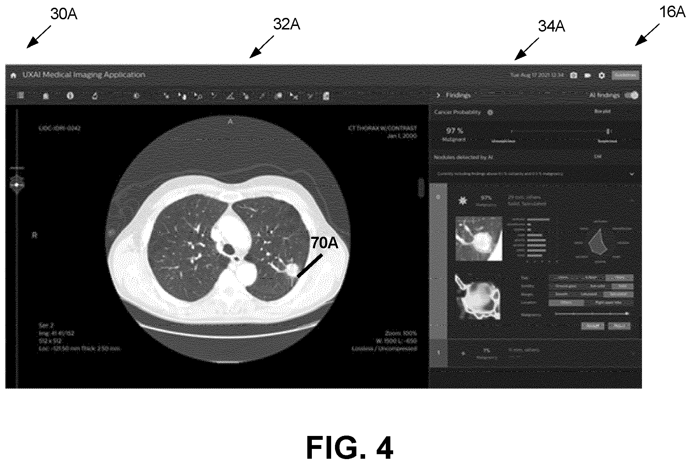

Based on the availability and richness of the AI results on nodule detection and assessment, and in some embodiments, the metrics related to AI robustness (e.g., confidence of predictions), the integration and representation of AI results is adapted (e.g., via the adaptive UI workflow component 22, Fig. 1). In other words, the AI results may be manifested as a primary or secondary reader to adapt the radiologist's workflow of image reading. Specifically, when AI has a high confidence of prediction, the AI works as the first reader, and directly shows the radiologist the findings, and leads to a quick sign-off and increases the efficiency of image reading. That is, the primary or first reader flow (more certainty) may be the result of instances where there is a very high likelihood of malignancy, very low AI uncertainty, and the derivation from the AI results of many nodule features (e.g. large, solid, spiculated nodule with irregular shape, etc.) that are indicative of a malignancy. The secondary reader (less certainty) flow may be the result of AI results corresponding to smaller sized nodules with a lower malignancy risk score and less suspicious nodule features (e.g., smooth margins). Based on the richness of AI results, there are two embodiments of the AI assessment panel 34 (also referred to herein simply as assessment panel 34) on the image reading page 16 for assisting nodule assessment. When AI has low confidence in malignancy risk prediction, the AI results are manifested in a secondary reader, where the AI panel is closed or hidden by default, which allows the radiologist to inspect the CT images first. Subsequently, the AI assessment panel 34 may be activated as a safety net to prevent the missed nodules. Figs. 4-6 illustrate several configurations of the image reading page 16 based on the metrics 28 and/or the richness or scope of the AI results 26.

Referring to Fig. 4, shown is an embodiment of an example image reading page 16A (similar to image reading page 16 of Fig. 1) according to a first configuration. For cases for which the AI model 18 (Fig. 1) generates rich nodule results with high robustness, the adaptive UI workflow component 22 (Fig. 1) adapts a first (e.g., reader) software flow, and the image reading page 16A is adapted as well. The image reading page 16A comprises the vertical UI component(s) 30A, the selected (e.g., from the UI component(s) 30A) image slice 32A, and the assessment panel 34A. The UI component(s) 30A (vertically oriented in this example, though not limited to that orientation) comprises the slider, markers, and area chart(s), as similarly described in association with Figs. 2A-2B. The image slice 32A comprises an identification 70A (also referred to as an identifying marker) of the nodule, which suggests or indicates size information, which in some instances may be based on segmentation of the nodule and in other cases, may corresponding to more limited features (and identified using a bounding box, cross, etc.). The assessment panel 34A presents rich AI results, similar to the information provided for the configuration described in association with Figs. 2A-2E, including nodule outline, characteristics, and malignancy prediction. In one embodiment, the information presented includes nodule features or characteristics, including size (e.g., size range as described above), solidity (e.g., ground-glass, sub-solid, solid), margin (e.g., smooth, lobulated, spiculated), location (e.g., right upper lobe, etc.), and/or malignancy risk (e.g., range based on percentages). Additional graphics and/or statistics pertaining to the AI results may also be presented, including a spider plot of nodule features, a bar chart of nodule features, and/or a grandcam visualization indicating which pixels contributed most/least to the likelihood of malignancy, as an illustrative example. Other and/or additional features of the image reading page 16A (and similar to the other reading pages 16B-16C), such as menu bars and user interactive features, may be presented, with the discussion of the same omitted here to avoid obscuring more relevant features of the image reading page 16A. Note that in some embodiments, some or all of the features of the nodule may be automatically derived by an algorithm which does not necessarily need to be AI.

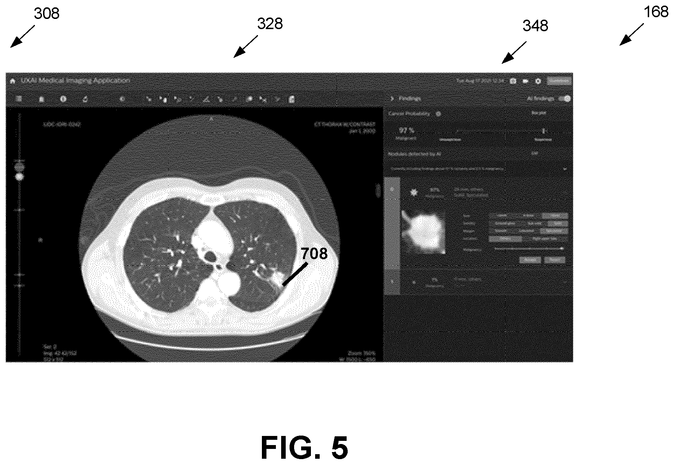

Fig. 5 shows an embodiment of an example image reading page 16B according to a second configuration. For cases for which the AI model 18 (Fig. 1) generates limited nodule results (e.g., depending on the richness or scope of the AI results, availability of AI results, etc.), yet high robustness, the adaptive UI workflow component 22 (Fig. 1) adapts a second software flow, and the image reading page 16B is adapted as well. The image reading page 16B comprises the vertical UI component(s) 30B, the selected (e.g., from the UI component(s) 30B) image slice 32B, and the assessment panel 34B. As explained above, when a user selects the visualization superimposed on the slider, the navigation tool navigates the user to the corresponding image slice (containing the nodule represented by the visualization) in the image adapted reading page and the assessment panel is opened along with the amplified or zoomed-in image of the nodule with metrics and/or characteristics of the nodule presented. The UI component(s) 30B comprises the slider 40, markers 42, and bubble charts 66 (or other types of visualizations in some embodiments), as similarly described in association with Figs. 3A-3B. The image slice 32B comprises an identification 70B (also referred to herein as an identifying marker) of the nodule, which suggests or indicates size information as similarly described above. The assessment panel 34B presents the AI results, which is of lesser scope than (e.g., a subset of) the information provided in the assessment panel 70A (Fig. 4), including nodule malignancy prediction and characteristics that are presented to the radiologists for nodule assessment. Note that the information presented in the assessment panel 34B may be more extensive (e.g., of a different richness or scope) than information suggested by the visualizations in the launched-from worklist (e.g., worklist 14). For instance, and as explained above, in the worklist, extensive information on the nodule characteristics is not necessarily required, since the slider may serve primarily to provide an impression to the user on the presence of a suspicious nodule and provide a navigation tool to open the image viewer (e.g., image reading page 16) for the nodule of interest. Subsequently, while in the image viewer, the user may desire more detailed information of the nodule to perform an evaluation.

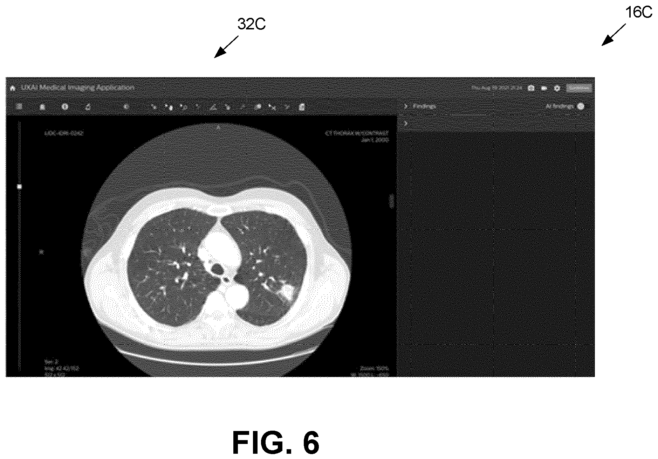

Fig. 6 shows an embodiment of an example image reading page 16C according to a third configuration. For cases for which the AI model 18 (Fig. 1) generates low certainty on its prediction (low robustness), the adaptive UI workflow component 22 (Fig. 1) adapts a third software flow, and the image reading page 16C is adapted as well. The image reading page 16C comprises no vertical UI component(s) or assessment panel, and simply presents an image slice 32C (with no identification). As described above, the AI results may be closed by default (hidden), yet optionally invoked or prompted after an initial assessment by the radiologist. Note that the assessment panel can comprise a combination of AI generated predictions and/or characterizations, and metrics derived from other algorithms (non-AI). For example, using a non-AI based segmentation algorithm, the nodule may be segmented from the image, after which the nodule density inside the segmented region can be calculated. In some embodiments, functionality for nodule detection may be performed using existing (non-AI) algorithms, and the malignancy prediction algorithm is AI-based.

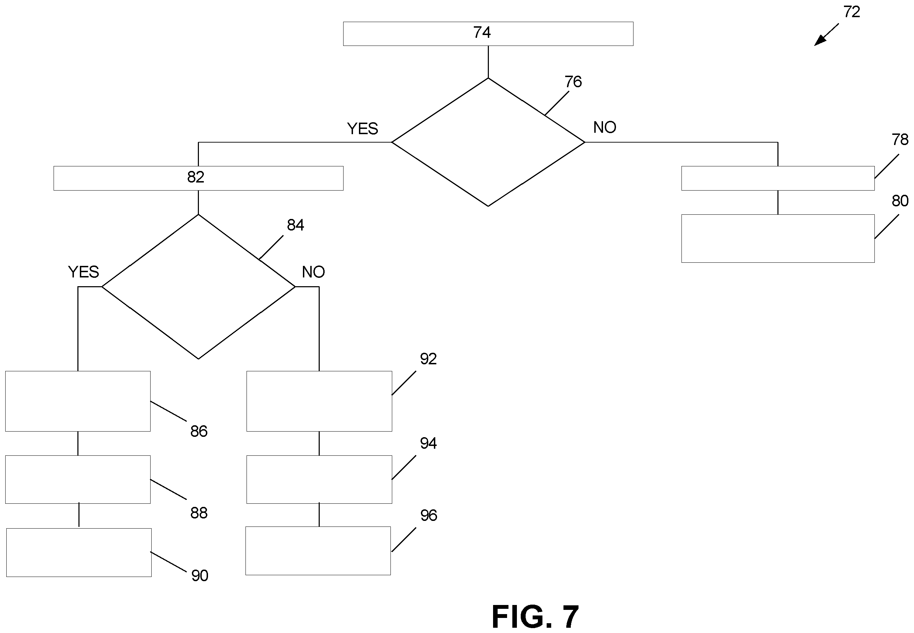

Fig. 7 shows an embodiment of an example adaptive workflow user interface method 72, in accordance with an embodiment of the invention. In one embodiment, the method 72 is performed by the CAD system 10, and in some embodiments, the CAD software 12. In general, the method 72 illustrates the adaptation of the user interfaces and interaction flow of CAD-supported radiology image reading software 12 based on the AI results. One goal of the method 72 is to improve radiologist-AI collaboration regarding both efficiency and accuracy. The method 72 includes receiving AI results pertaining to pulmonary nodule assessment (74). As explained in association with the CAD system 10, the AI results comprise the predicted results 26 (e.g., the location, size, severity, etc.) and the metrics 28 related to AI robustness and relevance (e.g., confidence of prediction). At (76), the method 72 determines whether the AI prediction has high robustness (e.g., greater than a threshold level, which may be determined based on the radiologist in a user-configuration screen, or preconfigured based on historical data, test or experimental results, simulations, protocols, etc.). If not ("no" for step 76), there is no navigation tool utilization in the worklist 14 (78), and the assessment panel 34 is closed (80), such as shown in Fig. 6. In some embodiments, the UI component(s) 30 may be hidden from view as a default, and later activated, as described above.

If the determination (76) is that the AI prediction has high robustness ("yes"), then the AI results are provided to the navigation tool 20 (82), and an additional determination is made by the method 72 as to whether the scope of the AI results are greater than (or in some embodiments, greater or equal to) a threshold (84). For instance, the threshold may correspond to whether the AI results are available or not (e.g., a binary decision). In some embodiments, the threshold may be based on a quantity (and/or quality) of information derived about nodule features or characteristics, which may be based on similar determinations as used in the threshold in step 76. If the threshold is exceeded (or in some embodiments, met or exceeded) ("yes"), the AI results are prepared for presentation (e.g., transformed) by the navigation tool 20 for AI results of a rich or extensive scope (86). The navigation tool 20 adapts the worklist 14 according to a rich information configuration (based on the AI results) using the UI component(s) 30A (88), such as illustrated in Figs. 2A-2B. The method 72 further includes the adaptive UI workflow component 22 adapting the image reading page 16 according to a rich information configuration, including the presentation of the assessment panel 34A (90), such as shown in Fig. 4.

If the determination (84) is that the scope of the AI results does not meet a threshold quantity and/or quality ("no"), then the AI results are prepared for presentation by the navigation tool 20 for AI results of a limited richness or scope (92). The navigation tool 20 adapts the worklist 14 according to a rich information configuration (based on the AI results) using the UI component(s) 30B (94), such as illustrated in Figs. 3A-3B. The method 72 further includes the adaptive UI workflow component 22 adapting the image reading page 16 according to a rich information configuration, including the presentation of the assessment panel 34 (96), such as shown in Fig. 5. Note that steps 82 and 86 (or 82 and 92) may differ in the sense that step 82 corresponds to predictions that only reflect the likelihood of the presence of a malignant nodule, whereas step 86 (or 92) corresponds to an assessment of the scope of nodule feature determinations. For instance, if the malignancy prediction shows high robustness, the AI results are provided to the navigation tool (e.g., step 82). If then also the AI results include information on nodule features (based on a determination at step 84), the results may be pushed to the navigation tool based on the scope (e.g., steps 86 or 92).

In effect, the robustness of AI prediction decides the visibility of AI results, which are provided by the navigation tool 20 in the worklist 14 and provided by the adaptive UI workflow component 22 in the assessment panel 34 in the imaging reading page 16. The richness of the AI prediction on nodule assessment is a basis for the navigation tool 20 and adaptive UI workflow component 22 to configure the worklist 14 and image reading page 16. Adapted by the AI results, there are potentially multiple image-reading environments for the radiologist to interpret the image for nodule assessment with full AI results, with part of AI results, or with no AI-generated output.

As noted in Fig. 7, when AI generates nodule predictions of high confidence, the AI findings are fully presented to the radiologist using the nodule navigation tool and the UI component(s) 30 in the worklist 14, and the adaptive UI workflow component 22 and assessment panel 34 on the image reading page 16. Based on the method 72 operations, AI can help the radiologist to make a quick sign-off and increase the efficiency of image reading. When AI has low confidence in prediction, the AI findings may be hidden at first, allowing the radiologist to inspect the CT images with their judgment, and then the AI findings may be activated as a safety-net to prevent missed nodules.

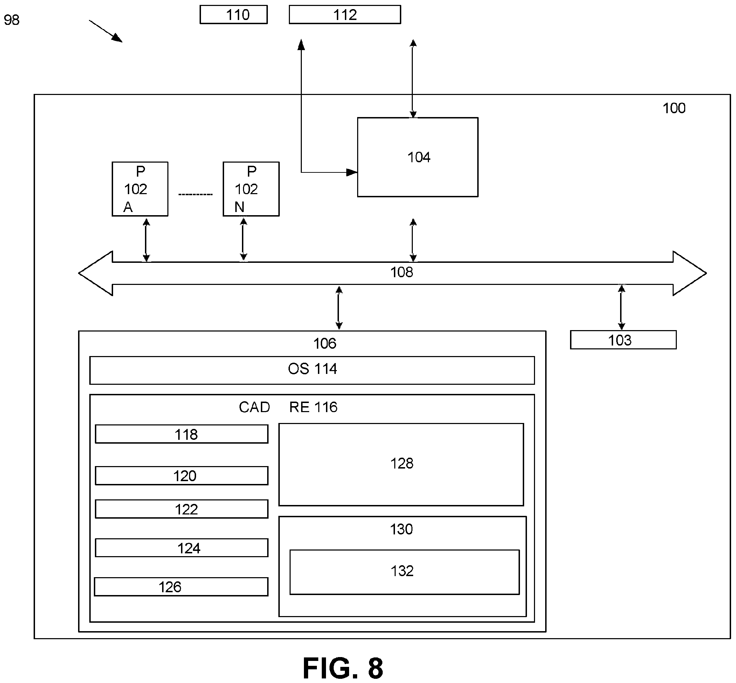

Referring now to Fig. 8, shown is an embodiment of an example computing system 98 that may be used to implement the CAD system 10 (and method 72). In the depicted embodiment, functionality of the CAD system 10 is implemented as a system of co-located software and hardware components collectively embodied as a computing device 100 (which may include a medical device) and plural sub-systems, wherein the computing device 100 is operatively connected to the plural sub-systems, as described below. In some embodiments, the computing device functionality may be embedded in one of the sub-systems, or one or more of the herein-described sub-system functionality may be integrated into fewer devices. It should be appreciated that, in some embodiments, functionality of the CAD system 10 may be implemented via plural computing devices that are networked at a similar location or situated in different locations, potentially remote from each other, and connected via one or more networks.

The computing device 100 includes one or more processors 102 (e.g., 102A...102N), input/output interface(s) 104, and memory 106, etc. coupled to one or more data busses, such as data bus 108. The processor(s) 102 may be embodied as a custom-made or commercially available processor, including a single or multi-core central processing unit (CPU), tensor processing unit (TPU), graphics processing unit (GPU), vector processing unit (VPU), or an auxiliary processor among several processors, a semiconductor-based microprocessor (in the form of a microchip), a macro-processor, one or more application specific integrated circuits (ASICs), field programmable gate arrays (FPGUs), a plurality of suitably configured digital logic gates, and/or other existing electrical configurations comprising discrete elements both individually and in various combinations to coordinate the overall operation of the computing device 100.

The I/O interfaces 104 comprise hardware and/or software to provide one or more interfaces to various sub-systems, including to one or more user interface(s) 110 and an imaging sub-system 112. The I/O interfaces 104 may also include additional functionality, including a communications interface for network-based communications. For instance, the I/O interfaces 104 may include a cable and/or cellular modem, and/or establish communications with other devices or systems via an Ethernet connection, hybrid/fiber coaxial (HFC), copper cabling (e.g., digital subscriber line (DSL), asymmetric DSL, etc.), using one or more of various communication protocols (e.g., TCP/IP, UDP, etc.). In general, the I/O interfaces 104, in cooperation with a communications module (not shown), comprises suitable hardware to enable communication of information via PSTN (Public Switched Telephone Networks), POTS, Integrated Services Digital Network (ISDN), Ethernet, Fiber, DSL/ADSL, Wi-Fi, cellular (e.g., 3G, 4G, 5G, Global System for Mobile Communications (GSM), General Packet Radio Service (GPRS),etc.), Bluetooth, near field communications (NFC), Zigbee, among others, using TCP/IP, UDP, HTTP, DSL.

The user interface(s) 110 may include a keyboard, scroll-wheel, mouse, microphone, immersive head set, display device(s), etc., which enable input and/or output by or to a user, and/or visualization to a user. In some embodiments, the user interface(s) 110 may cooperate with associated software to enable augmented reality or virtual reality. The user interface(s) 110, when comprising a display device, enables the display of segmented anatomical structure(s), including organs, nodules, tumor(s), abnormal growth tissue (e.g., collectively referred to herein as a region or regions of interest). In some embodiments, the user interface(s) 104 may be coupled directly to the data bus 108.

The imaging sub-system 110 includes one or more imaging sub-systems and/or image storage sub-systems that are used to enable visualization of anatomical structures and regions of interest. The imaging sub-system 110 may include ultrasound imaging, fluoroscopy, magnetic resonance, computed tomography, and/or positron emission tomography (PET). In some embodiments, the images may be retrieved from a picture archiving and communication systems (PACS) or any other suitable imaging component or delivery system. In some embodiments, the images provided by the imaging sub-system 110 may be segmented according to existing segmentation algorithms.

The memory 106 may include any one or a combination of volatile memory elements (e.g., random-access memory RAM, such as DRAM, and SRAM, etc.) and nonvolatile memory elements (e.g., ROM, Flash, solid state, EPROM, EEPROM, hard drive, tape, CDROM, etc.). The memory 106 may store a native operating system, one or more native applications, emulation systems, or emulated applications for any of a variety of operating systems and/or emulated hardware platforms, emulated operating systems, etc. In some embodiments, a separate storage device (STOR DEV, 103) may be coupled to the data bus 108 or as a network-connected device (or devices) via the I/O interfaces 104 and one or more networks. The storage device may be embodied as persistent memory (e.g., optical, magnetic, and/or semiconductor memory and associated drives).

In the embodiment depicted in Fig. 8, the memory 106 comprises an operating system 114 (OS) (e.g., LINUX, macOS, Windows, etc.), and CAD software 116. In one embodiment, the CAD software 116 includes the functionality shown in and described in association with Figs. 1 and 7, and comprises a plurality of modules (e.g., executable code) co-hosted on the computing device 100, though in some embodiments, the modules may be distributed across various systems or sub-systems over one or more networks, including implementation using a cloud computing platform. The CAD software 116 comprises a segmentation module 118, an AI model 120, a worklist module 122, a navigation tool 124 comprising a UI component module 126, an image reading page module 128, and an adaptive UI workflow module 130 comprising an assessment panel module 132. Note that functionality of the modules of the CAD software 116 may be shared amongst each other. In some embodiments, there may be fewer or additional modules. For instance, functionality of some modules may be combined, or additional functionality may be implemented yet not shown, such as a communication module, security module, etc.

Functionality of one or more of the various modules is briefly explained here, with further description below. The segmentation module 118 provides image segmentation of anatomical structures according to existing segmentation functionality. In some embodiments, segmentation may be performed elsewhere, and the segmented imaging transferred to the CAD software 116.

The AI model 120 may utilize any of existing AI functionality that creates, trains, and deploys machine learning algorithms that emulate logical decision making, and includes linear or logical regression algorithms, decision trees, Bayes, K-nearest neighbors, support vector machines, and/or neural or deep neural networks.

The worklist module 122 and image reading page module 128 may be included in existing CAD-supported image reading software, and provide for the formatting and rendering (in a graphical user interface) of the worklists (e.g., worklist 14) and the image reading pages (e.g., image reading page 16). The navigation tool 124 includes the UI component module 126, the navigation tool 124 (e.g., a model) transforming the AI results into elements of the UI component(s) 30 for adaptive presentation within the worklist 14 and for the activation of interactive user interface elements, including thumbnails (e.g., thumbnails 52A or 52B) based on AI result confidence and richness. The adaptive UI workflow module 130 includes the assessment panel module 132, which prepares and renders the AI results for presentation of the assessment panel 34, identification within the image of ROIs, and the presentation of the UI component(s) 30 (e.g., in cooperation with the navigation tool 124) based on AI result confidence and richness.

Note that the memory 106 and storage device may each be referred to herein as a non-transitory, computer readable storage medium or the like.

Execution of the CAD software 116 may be implemented by the one or more processors 102 under the management and/or control of the operating system 114.

When certain embodiments of the computing device 100 are implemented at least in part with software (including firmware), it should be noted that the CAD software 116 can be stored on a variety of non-transitory computer-readable (storage) medium for use by, or in connection with, a variety of computer-related systems or methods. In the context of this document, a computer-readable medium may comprise an electronic, magnetic, optical, or other physical device or apparatus that may contain or store a computer program (e.g., executable code or instructions) for use by or in connection with a computer-related system or method. The software may be embedded in a variety of computer-readable mediums for use by, or in connection with, an instruction execution system, apparatus, or device, such as a computer-based system, processor-containing system, or other system that can fetch the instructions from the instruction execution system, apparatus, or device and execute the instructions. Note that some embodiments may implement functionality of the computing device 100 via a cloud computing platform.

When certain embodiments of the computing device 100 are implemented at least in part with hardware, such functionality may be implemented with any or a combination of the following technologies, which are all already existing in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), TPUs, GPUs, and/or other accelerators/co-processors, etc.

One having ordinary skill in the art should appreciate in the context of the present disclosure that the example computing device 100 is merely illustrative of one embodiment, and that some embodiments of computing devices may comprise fewer or additional components, and/or some of the functionality associated with the various components depicted in Fig. 8 may be combined, or further distributed among additional modules or computing devices, in some embodiments. It should be appreciated that certain well-known components of computer systems are omitted here to avoid obfuscating more relevant features of the computing device 100.

Though emphasis is placed herein on the decision of whether to provide the AI results based on the reliability metric, followed by (when deciding to present the AI results) adaptation of certain user interfaces based on a richness or scope of information, it should be appreciated by one having ordinary skill in the art in the context of the present disclosure that variations to the disclosed embodiments are contemplated to be within the scope of the disclosure. For instance, in one embodiment, information is received for a region of interest located in an image slice of an image stack, a first user interface and a user interface (UI) component(s) is provided, the UI component(s) comprising a slider and one or more visualizations suggestive of features of the region of interest corresponding to the information, the slide corresponding to the image stack, and based on a first input associated with the one or more visualizations, navigating to a second user interface that is further descriptive of the region of interest. In one embodiment, the information comprises AI results. In one embodiment, the information comprises user-inputted information. In one embodiment, the first user interface and the second user interface are adapted according to one among a plurality of configurations, where in the configurations are different based on a scope of the information. In some embodiments, the configuration may be based on metrics associated with the information, and in some embodiments, the configuration may be based regardless of metrics associated with the information.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Note that various combinations of the disclosed embodiments may be used, and hence reference to an embodiment or one embodiment is not meant to exclude features from that embodiment from use with features from other embodiments. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical medium or solid-state medium supplied together with or as part of other hardware, but may also be distributed in other forms. Any reference signs in the claims should be not construed as limiting the scope.

## Claims

1. A system (98), comprising:
a memory (106) comprising instructions (116); and
one or more processors (102) configured to carry out the instructions, which cause the system (98) to:
receive Artificial Intelligence ,AI, results of an applied AI model, wherein the AI results are related to a region of interest;
receive a reliability metric associated with the AI results; and
determin whether to provide the AI results or hide the AI results based on the reliability metric.

2. The system of claim 1, wherein the one or more processors are further configured to hide the AI results based on a comparison of the reliability metric to a pre-defined threshold.

3. The system of any one of the preceding claims, wherein the one or more processors are configured to adapt a first user interface (14A) according to a configuration of an interactive user interface component (30A) selected from among plural configurations based on a scope of the AI results, wherein the user interface component comprises:
a slider (40) representing plural imaging slices, a marker (42) corresponding to a location among the plural imaging slices of the region of interest, and a visualization (44) superimposed on the marker (42), and that is visually suggestive of characteristics of the region of interest from the AI results.

4. The system of any one of the preceding claims, wherein a quantity of the characteristics suggested by the visualization differs among the plural configurations.

5. The system of any one of the preceding claims, wherein the visualization differs among the plural configurations.

6. The system of any one of the preceding claims, wherein the one or more processors are further configured to provide a thumbnail image (52A) based on user input over the marker, the thumbnail image comprising an image (54) of the region of interest, an identifying marker (56) of the region of interest within an anatomical structure, and a visual cue (58) suggestive of characteristics of the region of interest.

7. The system of any one of the preceding claims, wherein the one or more processors are further configured to determine whether to provide the AI results in a second user interface or to hide the AI results in the second user interface based on the reliability metric.

8. The system of any one of the preceding claims, wherein the one or more processors are further configured to navigate to the second user interface (16) based on user selection of a user interface component in a first user interface, wherein the one or more processors are further configured to adapt the second user interface according to one of plural assessment user interface configurations based on a scope of the AI results.

9. The system of any one of the preceding claims, wherein the second user interface comprises an image reading page (16A) that includes an image involving the region of interest, wherein the one or more processors are configured to adapt the second user interface according to a first assessment user interface configuration selected from among the plural assessment user interface configurations based on a first scope among plural scopes of the AI results, wherein the first assessment user interface configuration further comprises:
an identifying marker (70A) of the region of interest within an anatomical structure provided in the image; and
a first assessment panel (34A) adjacent to the image, the first assessment panel comprising a first set of information corresponding to the first scope that is of greater scope than a rest of the plural scopes.

10. The system of any one of the preceding claims, wherein the second user interface comprises an image reading page that includes an image involving the region of interest,, wherein the one or more processors are configured to adapt the second user interface according to a second assessment user interface configuration selected from among the plural assessment user interface configurations based on a second scope among plural scopes of the AI results, wherein the second assessment user interface configuration further comprises:
an identifying marker (70B) of the region of interest within an anatomical structure provided in the image; and
a second assessment panel (34B) adjacent to the image, the second assessment panel comprising a second set of information corresponding to the second scope.

11. The system of any one of the preceding claims, wherein the second user interface comprises of an image reading page that includes an image involving the region of interest,, wherein the one or more processors are configured to adapt the second user interface according to a third assessment user interface configuration selected from among the plural assessment user interface configurations based on a comparison of the reliability metric to a threshold, wherein the third assessment user interface does not show as a default an identifying marker of the region of interest within an anatomical structure provided in the image nor a corresponding assessment panel.

12. The system of any one of the preceding claims, wherein the system comprises a cloud computing platform.

13. A computer-implemented method (72) configured to be carried out by the system of any one of claims 1-12.

14. A non-transitory, computer readable storage medium (106) comprising instructions (116) which, when executed by the system of claims 1-12, cause the system to carry out the steps of the method of claim 13.

15. A computer program comprising instructions which, when the program is executed by a computer, cause the computer to carry out the method of claim 13.
